# EUROPEAN PATENT SPECIFICATION

(11) **EP 0 684 982 B1**
(45) Date of publication and mention of the grant of the patent: **28.08.2002**
(21) Application number: 94907403.3
(22) Date of filing: 01.02.1994
(51) Int. Cl.: C11D 1/66, C11D 1/72, C11D 1/83, C11D 1/94, C11D 3/16, C11D 3/46, A61K 7/50

(54) **CLEANSING COMPOSITIONS**
KÖRPERPFLEGEMITTEL
COMPOSITIONS D'HYGIENE CORPORELLE

(30) Priority: 11.02.1993 GB 9302710
(43) Date of publication of application: 06.12.1995
(73) Proprietor: THE PROCTER & GAMBLE COMPANY, Cincinnati, Ohio 45202 (US)
(72) Inventor: LEAHY, Christopher, David 17 North Road, Richmond Surrey TW9 4HA (GB); RICHARDSON, Wendy, Victoria, Jane, Staines Middlesex TW18 4EZ (GB); DUKE, Roland, Philip, Berkshire RG11 2NB (GB)
(74) Representative: McKinney, Victoria
(86) International application number: US9401165
(87) International publication number: WO94018292

(56) References cited:
- EP-A- 0 160 269
- EP-A- 0 485 212
- EP-A- 0 486 074
- WO-A-93/09761
- US-A- 4 247 425
- US-A- 4 256 611
- US-A- 4 338 211
- US-A- 4 343 726
- US-A- 4 603 005
- US-A- 4 946 670
- US-A- 5 000 868
- US-A- 5 013 473
- US-A- 5 179 128

## Description

### TECHNICAL FIELD

The present invention relates to cleansing compositions. In particular it relates to mild personal cleansing compositions with good skin feel attributes and foaming properties suitable for simultaneously cleansing and conditioning the skin and which may be used, for example, in the form of foam bath preparations, shower products, skin cleansers, hand, face and body cleansers, etc.

### BACKGROUND OF THE INVENTION

Mild cosmetic compositions must satisfy a number of criteria including cleansing power, foaming properties and mildness/low irritancy/good feel with respect to the skin, and the ocular mucosae. Skin is made up of several layers of cells which coat and protect the keratin and collagen fibrous proteins that form the skeleton of its structure. The outermost of these layers, referred to as the stratum corneum, is known to be composed of 250 A protein bundles surrounded by 80 Å thick layers. Anionic surfactants can penetrate the stratum corneum membrane and the cuticle and, by delipidization destroy membrane integrity. This interference with skin protective membranes can lead to a rough skin feel and eye irritation and may eventually permit the surfactant to interact with the keratin creating irritation and loss of barrier and water retention functions.

Ideal cosmetic cleansers should cleanse the skin gently, without defatting and/or drying the skin and without irritating the ocular mucosae or leaving skin taut after frequent use. Most lathering soaps, bath products, and bars fail in this respect. US-A-4,343,726 discloses a detergent composition having low eye and skin irritation properties. WO-A-93/09761, a document falling within the terms of Art. 54(3) EPC, discloses compositions which provide both a skin cleansing and skin moisturizing benefit from the same product.

Certain synthetic surfactants are known to be mild. However, a major drawback of most mild synthetic surfactant systems when formulated for shampooing or personal cleansing is poor lather performance compared to the highest shampoo and bar soap standards. Thus, surfactants that are among the mildest, such as sodium lauryl glyceryl ether sulfonate, (AGS), are marginal in lather. The use of known high sudsing anionic surfactants with lather boosters, on the other hand, can yield acceptable lather volume and quality but at the expense of clinical skin mildness. These two facts make the surfactant selection, the lather and mildness benefit formulation process a delicate balancing act.

Despite the many years of research that have been expended by the toiletries industry on personal cleansing, the broad mass of consumers remain dissatisfied by the mildness of present day cleansing compositions, finding, for example, that they have to apply a separate cosmetic lotion or cream moisturizer to the skin after using a shower or bath preparation in order to maintain skin suppleness and hydration and to counteract the delipidizing effect of the cleanser.

It has now been found that personal cleansing compositions having improved skin feel attributes both for in use feel and after use feel and product stability can be formed by the use of certain oil derived nonionic surfactants in combination with water soluble citrate salts.

Thus a need exists for personal cleansing products which will not dehydrate the skin or result in loss of skin suppleness, which will provide a level of skin conditioning performance in a wash and rinse-off product which previously has only been provided by a separate post-cleansing cosmetic moisturizer and which will produce a foam which is stable and of high quality, which are effective skin cleansers, which have good rinsibility characteristics, and which at the same time have stable product and viscosity characteristics and remain fully stable under long term and stressed temperature storage conditions.

### SUMMARY OF THE INVENTION

The subject of the present invention is a mild, foam-producing cleansing product suitable for personal cleansing of the skin and which may be used as foam bath and shower products, skin cleansers etc. According to one aspect of the invention, there is provided a detergent, personal cleansing or cosmetic composition for personal cleansing of the skin comprising:
(a) from 10% to 50% by weight of one or more surfactants selected from anionic, zwitterionic and/or amphoteric surfactants and mixtures thereof;
(b) from 0.1% to 20% by weight of a soluble or dispersible nonionic surfactants selected from ethoxylated animal and vegetable oils and fats;
(c) from 1.5% to 10% by weight of citric acid or water soluble citrate salt or mixture thereof;
(d) up to 3% by weight of perfume or cosmetic oil; and
(e) water.

In a highly preferred embodiment, the invention takes the form of a foam producing cleansing composition with superior skin feel characteristics, improved perceived dryness and assessed tightness and expertly graded dryness, combined with excellent lathering, good stability, cleansing ability and conditioning performance.

All concentrations and ratios herein are by weight of the cleansing composition, unless otherwise specified. Surfactant chain lengths are also on a weight average chain length basis, unless otherwise specified.

The cleansing compositions herein are based on a combination of citrate salt and mild surfactants, which in general terms can be selected from anionic, amphoteric and zwitterionic surfactants and mixtures thereof, together with certain nonionic surfactants derived from vegetable and animal oils and fats. The total level of surfactant, inclusive of anionic, oil derived nonionic, zwitterionic, amphoteric and other surfactant components is preferably from 5% to 60%, more preferably from 8% to 40%, and especially from 10% to 35% by weight. The compositions preferably comprise a mixture of anionic with zwitterionic and/or amphoteric surfactants. The level of each of the anionic surfactant and zwitterionic and/or amphoteric surfactant is in the range from 1% to 15%, and especially from 2% to 13% by weight of the composition, while the level of oil-derived nonionic surfactant is preferably from 2% to 16%, more preferably from 3% to 12% by weight. The total level of anionic, amphoteric, and zwitterionic surfactant components, is preferably from 10% to 50%, more preferably from 10% to 35% and especially from 10% to 30% by weight of composition. The weight ratio of anionic surfactant : zwitterionic and/or amphoteric surfactant is in the range from 1:2 to 6:1. Other suitable compositions within the scope of the invention comprise mixtures of anionic and zwitterionic and/or amphoteric with one or more auxillary nonionic surfactants or mixtures thereof. The level of the auxiliary nonionic surfactant is in the range from 0.1 % to 20% by weight of the composition. Further suitable compositions within the scope of the invention comprise mixtures of oil derived nonionic surfactants, cosmetic oils and anionic, zwitterionic and/or amphoteric surfactants, wherein the level of cosmetic oil is up to 3% by weight of the composition and the weight ratio of oil derived nonionic surfactant:cosmetic oil is at least 1:2. Compositions according to the invention also include citric acid or water soluble citrate salt or mixture thereof at levels from 1.5% to 10% by weight wherein the weight ratio of oil derived nonionic surfactant: citric acid or water soluble salt thereof is preferably in the range from 1:2 to 3:1

Anionic surfactants suitable for inclusion in the compositions of the invention can generally be described as mild synthetic detergent surfactants and include ethoxylated alkyl sulfates, alkyl glyceryl ether sulfonates, methyl acyl taurates, fatty acyl glycinates, N-acyl glutamates, acyl isethionates, alkyl sulfosuccinates, alpha-sulfonated fatty acids, their salts and/or their esters, alkyl ethoxy carboxylates, alkyl phosphate esters, ethoxylated alkyl phosphate esters, acyl sarcosinates and fatty acid/protein condensates, and mixtures thereof. Alkyl and/or acyl chain lengths for these surfactants are C₈-C₂₂, preferably C₁₀-C₁₈.

Preferred for use herein from the viewpoint of optimum mildness and lathering characteristics are the salts of sulfuric acid esters of the. reaction product of 1 mole of a higher fatty alcohol and from about 1 to about 12 moles of ethylene oxide, with sodium and magnesium being the preferred counterions. Particularly preferred are the alkyl sulfates containing from about 2 to 6, preferably 2 to 4 moles of ethylene oxide, such as sodium laureth-2 sulfate, sodium laureth-3 sulfate and magnesium sodium laureth-3.6 sulfate. In preferred embodiments, the anionic surfactant contains at least 50%, especially at least 75% by weight of ethoxylated alkyl sulfate.

The compositions for use herein suitably also contain an amphoteric surfactant. Amphoteric surfactants suitable for use in the compositions of the invention include:
(a) imidazolinium surfactants of formula (I) 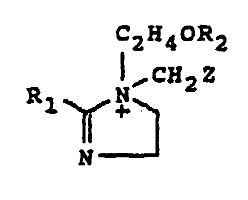 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (II) 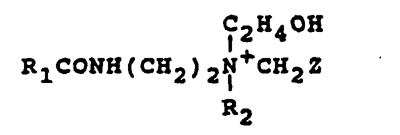 wherein R₁, R₂ and Z are as defined above;
(b) aminoalkanoates of formula (III)

   R₁NH(CH₂)ₙCO₂M

   and iminodialkanoates of formula (IV)

   R₁N[(CH₂)ₘCO₂M]₂

   wherein n and m are numbers from 1 to 4, and R₁ and M are independently selected from the groups specified above; and
(c) mixtures thereof.

Suitable amphoteric surfactants of type (a) are marketed under the trade name Miranol and Empigen and are understood to comprise a complex mixture of species. Traditionally, the Miranols have been described as having the general formula I, although the CTFA Cosmetic Ingredient Dictionary, 4th Edition indicates the non-cyclic structure II. In practice, a complex mixture of cyclic and non-cyclic species is likely to exist and both definitions are given here for sake of completeness. Preferred for use herein, however, are the non-cyclic species.

Examples of suitable amphoteric surfactants of type (a) include compounds of formula I and/or II in which R₁ is C₈H₁₇ (especially iso-capryl), C₉H₁₉ and C₁₁H₂₃ alkyl. Especially preferred are the compounds in which R₁ is C₉H₁₉, Z is CO₂M and R₂ is H; the compounds in which R₁ is C₁₁H₂₃, Z is CO₂M and R₂ is CH₂CO₂M; and the compounds in which R₁ is C₁₁H₂₃, Z is CO₂M and R₂ is H.

In CTFA nomenclature, materials preferred for use in the present invention include cocoamphocarboxypropionate, cocoamphocarboxy propionic acid, and especially cocoamphoacetate and cocoamphodiacetate (otherwise referred to as cocoamphocarboxyglycinate). Specific commercial products include those sold under the trade names of Empigen CDL60 and CDR 60 (Albright & Wilson), Miranol H2M Conc. Miranol C2M Conc. N.P., Miranol C2M Conc. O.P., Miranol C2M SF, Miranol CM Special (Rhone-Poulenc); Alkateric 2CIB (Alkaril Chemicals); Amphoterge W-2 (Lonza, Inc.); Monateric CDX-38, Monateric CSH-32 (Mona Industries); Rewoteric AM-2C (Rewo Chemical Group); and Schercotic MS-2 (Scher Chemicals).

It will be understood that a number of commercially-available amphoteric surfactants of this type are manufactured and sold in the form of electroneutral complexes with, for example, hydroxide counterions or with anionic sulfate or sulfonate surfactants, especially those of the sulfated C₈-C₁₈ alcohol, C₈-C₁₈ ethoxylated alcohol or C₈-C₁₈ acyl glyceride types. Preferred from the viewpoint of mildness and product stability, however, are compositions which are essentially free of (non-ethoxylated) sulfated alcohol surfactants. Note also that the concentrations and weight ratios of the amphoteric surfactants are based herein on the uncomplexed forms of the surfactants, any anionic surfactant counterions being considered as part of the overall anionic surfactant component content.

Examples of suitable amphoteric surfactants of type (b) include salts, especially the triethanolammonium salts and salts of N-lauryl-beta-amino propionic acid and N-lauryl-imino-dipropionic acid. Such materials are sold under the trade name Deriphat by Henkel and Mirataine by Rhône-Poulenc. Amphoterics preferred for use herein, however, are those of formula I and/or II.

The compositions of the invention also contain from 0.1% to 20%, preferably from 1% to 15%, and more preferably from 2% to 10% by weight of an oil derived nonionic surfactant or mixture of oil derived nonionic surfactants. The nonionic surfactants for use herein are soluble or dispersible nonionic surfactants selected from ethoxylated animal and vegetable oils and fats and mixtures thereof. Oil derived nonionic surfactants are valuable in compositions according to the invention for the provision of skin feel benefits both in use and after use. Suitable oil derived nonionic surfactants for use herein can be selected from water soluble vegetable and animal-derived emollients such as triglycerides with a polyglycol chain inserted; ethoxylated mono and di-glycerides, polyethoxylated lanolins and shea butter derivatives. One preferred class of oil-derived nonionic surfactants for use herein have the general formula (V) 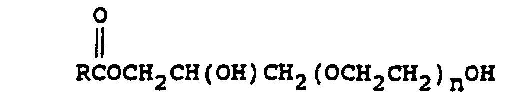 wherein n is from 5 to 200, preferably from 20 to 100, more preferably from 30 to 85, and wherein R comprises an aliphatic radical having on average from 5 to 20 carbon atoms, preferably from 9 to 18 carbon atoms.

Suitable ethoxylated oils and fats of this class include polyethyleneglycol derivatives of glyceryl cocoate, glyceryl caproate, glyceryl caprylate, glyceryl tallowate, glyceryl palmate, glyceryl stearate, glyceryl laurate, glyceryl oleate, glyceryl ricinoleate, and glyceryl fatty esters derived from triglycerides, such as palm oil, almond oil, and corn oil, preferably glyceryl tallowate and glyceryl cocoate.
Suitable oil derived nonionic surfactants of this class are available from Croda Inc. (New York, USA) under their Crovol line of materials such as Crovol EP40 (PEG 20 evening primrose glyceride), Crovol EP 70 (PEG 60 evening primrose glyceride) Crovol A-40 (PEG 20 almond glyceride), Crovol A-70 (PEG 60 almond glyceride), Crovol M-40 (PEG 20 maize glyceride), Crovol M-70 (PEG 60 maize glyceride), Crovol PK-40 (PEG 12 palm kernel glyceride), and Crovol PK-70 (PEG 45 palm kernel glyceride) and under their Solan range of materials such as Solan E, E50 and X polyethoxylated lanolins. Further suitable surfactants of this class are commercially available from Sherex Chemical Co. (Dublin, Ohio, USA) under their Varonic LI line of surfactants. These include, for example, Varonic LI 48 (polyethylene glycol (n=80) glyceryl tallowate, alternatively referred to as PEG 80 glyceryl tallowate), Varonic LI 2 (PEG 28 glyceryl tallowate), Varonic LI 420 (PEG 200 glyceryl tallowate), and Varonic LI 63 and 67 (PEG 30 and PEG 80 glyceryl cocoates). Other water soluble vegetable-derived emollients suitable for use are PEG derivatives of corn, avocado and babassu oil.

Also suitable for use herein are nonionic surfactants derived from composite vegetable fats extracted from the fruit of the Shea Tree (Butyrospermum Karkii Kotschy) and derivatives thereof. This vegetable fat, known as Shea Butter is widely used in Central Africa for a variety of means such as soap making and as a barrier cream, it is marketed by Sederma (78610 Le Perray En Yvelines, France). Also of interest are ethoxylated derivatives of Shea butter available from Karlshamn Chemical Co. (Columbos, Ohio, USA) under their Lipex range of chemicals, such as Lipex 102 E-75 (ethoxylated mono, di-glycerides of Shea butter). Similarly, ethoxylated derivatives of Mango, Cocoa and Illipe butter may be used in compositions according to the invention. Although these are classified as ethoxylated nonionic surfactants it is understood that a certain proportion may remain as non-ethoxylated vegetable oil or fat.

Other suitable oil-derived nonionic surfactants include ethoxylated derivatives of almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazlenut oil, olive oil, grapeseed oil, and sunflower seed oil.

Oil derived nonionic surfactants highly preferred for use herein from the viewpoint of optimum mildness and skin feel characteristics are PEG 60 evening primrose triglycerides; PEG 55 lanolin polyethoxylated derivatives and ethoxylated derivatives of Shea butter.

In addition to the above oil derived nonionic surfactants, the compositions of the invention can also comprise an auxiliary nonionic surfactant at levels from 0.1 % to 20%, more preferably from 0.1% to 10%, and especially from 1% to 8% by weight. Surfactants of this class include C12-C14 fatty acid mono-and diethanolamides, sucrose polyester surfactants and polyhydroxy fatty acid amide surfactants having the general formula (VI).

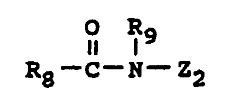

The preferred polyhydroxy fatty acid amide surfactants are those in which R₉ is C₁₋₄ alkyl, preferably methyl, and R₈ is C₇-C₁₉ alkyl or alkenyl, more preferably straight-chain C₉-C₁₇ alkyl or alkenyl, or mixture thereof; and Z₂ is a polyhydroxyhydrocarbyl having a linear hydrocarbyl chain with at least 3 hydroxyls directly connected to the chain, or an alkoxylated derivative (preferably ethoxylated or propoxylated) thereof. Z₂ preferably will be derived from a reducing sugar in a reductive amination reaction; more preferably Z₂ is a glycityl. Suitable reducing sugars include glucose, fructose, maltose, lactose, galactose, mannose, and xylose. As raw materials, high dextrose corn syrup, high fructose corn syrup, and high maltose corn syrup can be utilized as well as the individual sugars listed above. These corn syrups may yield a mix of sugar components for Z₂. It should be understood that it is by no means intended to exclude other suitable raw materials. Z₂ preferably will be selected from the group consisting of - CH₂(CHOH)ₙ-CH₂OH,-CH(CH₂OH)-(CHOH)ₙ₋₁-CH₂OH, -CH₂-(CHOH)₂(CHOR')(CHOH)-CH₂OH, where n is an integer from 3 to 5, inclusive, and R' is H or a cyclic or aliphatic monosaccharide, and alkoxylated derivatives thereof. Most preferred are glycityls wherein n is 4, particularly -CH₂-(CHOH)₄-CH₂OH.

The most preferred polyhydroxy fatty acid amide has the formula R₈(CO)N(CH₃)CH₂(CHOH)₄CH₂OH wherein R₈ is a C11-C17 straight chain alkyl or alkenyl group.

The compositions herein preferably also contain from 0.1% to 20%, more preferably from 0.1% to 10%, and especially from 1% to 8% of a zwitterionic surfactant.

Betaine surfactants suitable for inclusion in the composition of the invention include alkyl betaines of the formula R₅R₆R₇N⁺(CH₂)ₙM (VII) and amido betaines of the formula (VIII) 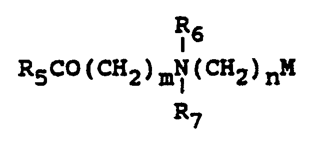 wherein R₅ is C₁₂-C₂₂ alkyl or alkenyl, R₆ and R₇ are independently C₁-C₃ alkyl, M is H, alkali metal, alkaline earth metal, ammonium or alkanolammonium, and n, m are each numbers from 1 to 4. Preferred betaines include cocoamidopropyldimethylcarboxymethyl betaine, laurylamidopropyldimethylcarboxymethyl betaine and Tego betaine.

Another essential component of the compositions of the invention is citric acid or water soluble citrate salt or mixtures thereof, expecially Na, K, NH₄ salts, at a level from 1.5% to 10% by weight preferably from 2% to 8% by weight, most preferably from 2% to 6% by weight. Citrate is important for providing improved physical and viscosity characteristics as well as enhanced skin feel properties in combination with the oil-derived nonionic components.

The compositions of the invention may also include an insoluble perfume or cosmetic oil or wax or a mixture thereof at a level up to 3 % by weight wherein the oil or wax is insoluble in the sense of being insoluble in the product matrix at a temperature of 25°C. Addition of such oils or waxes can provide emolliency, mildness and rinsibility characteristics to personal cleansing compositions according to the invention. It is a feature of the invention, however, that compositions having excellent emolliency and mildness together with desirable physical attributes (clarity etc.) can be delivered which are essentially oil-free, ie which contain less than 1%, preferably less than 0.5% by weight of an added oil phase. Physically, preferred compositions of this type take the form of an optically-clear solution or microemulsion. In compositions including an additional perfume or cosmetic oil or wax, preferably the weight ratio of oil-derived nonionic surfactant to added oil is at least 1:2, more especially at least 3:1.

Suitable insoluble cosmetic oils and waxes for use herein can be selected from water-insoluble silicones inclusive of non-volatile polyalkyl and polyaryl siloxane gums and fluids, volatile cyclic and linear polyalkylsiloxanes, polyalkoxylated silicones, amino and quaternary ammonium modified silicones, rigid cross-linked and reinforced silicones and mixtures thereof, C₁-C₂₄ esters of C₈-C₃₀ fatty acids such as isopropyl myristate and cetyl ricinoleate, beeswax, saturated and unsaturated fatty alcohols such as behenyl alcohol, hydrocarbons such as mineral oils, petrolatum and squalene, fatty sorbitan esters (see US-A-3988255, Seiden, issued October 26th 1976), lanolin and oil-like lanolin derivatives, animal and vegetable triglycerides such as almond oil, peanut oil, wheat germ oil, linseed oil, jojoba oil, oil of apricot pits, walnuts, palm nuts, pistachio nuts, sesame seeds, rapeseed, cade oil, corn oil, peach pit oil, poppyseed oil, pine oil, castor oil, soybean oil, avocado oil, safflower oil, coconut oil, hazlenut oil, olive oil, grapeseed oil, and sunflower seed oil, and C₁-C₂₄ esters of dimer and trimer acids such as diisopropyl dimerate, diisostearylmalate, diisostearyldimerate and triisostearyltrimerate.

The compositions of the invention may also contain a cationic or nonionic polymeric skin conditioning agent at a level from 0.01% to 5%, preferably from about 0.04% to 2% and especially from 0.05% to 1%. The polymer is found to be valuable for enhancing the creaminess and quality of the foam as well as providing a skin conditioning utility.

Suitable polymers are high molecular weight materials (mass-average molecular weight determined, for instance, by light scattering, being generally from about 2,000 to about 3,000,000, preferably from about 5,000 to about 1,000,000).

Useful polymers are the cationic, nonionic, amphoteric, and anionic polymers useful in the cosmetic field. Preferred are cationic and nonionic polymers used in the cosmetic fields as hair or skin conditioning agents.

Representative classes of polymers include cationic and nonionic polysaccharides; cationic and nonionic homopolymers and copolymers derived from acrylic and/or methacrylic acid; cationic and nonionic cellulose resins; cationic copolymers of dimethyldiallylammonium chloride and acrylic acid; cationic homopolymers of dimethyldiallylammonium chloride; cationic polyalkylene and ethoxypolyalkylene imines; quaternized silicones, and mixtures thereof.

By way of exemplification, cationic polymers suitable for use herein include cationic guar gums such as hydroxypropyl trimethyl ammonium guar gum (d.s. of from 0.11 to 0.22) available commercially under the trade names Jaguar C-14-S(RTM) and Jaguar C-17(RTM) and also Jaguar C-16(RTM), which contains hydroxypropyl substituents (d.s. of from 0.8-1.1) in addition to the above-specified cationic groups, and quaternized cellulose ethers available commercially under the trade name Ucare Polymer JR and Celquat. Other suitable cationic polymers are homopolymers of dimethyldiallylammonium chloride available commercially under the trade name Merquat 100, copolymers of dimethy aminoethylmethacrylate and acrylamide, copolymers of dimethyldiallylammonium chloride and acrylamide, available commercially under the trade names Merquat 550 and Merquat S, quaternized vinyl pyrrolidone acrylate or methacrylate copolymers of amino alcohol available commercially under the trade name Gafquat, and polyalkyleneimines such as polyethylenimine and ethoxylated polyethylenimine.

Anionic polymers suitable herein include hydrophobically-modified cross-linkers polymers of acrylic acid having amphipathic properties as marketed by B F Goodrich under the trade name Pemulen TRI and Pemulen TR2; and the carboxyvinyl polymers sold by B F Goodrich under the trade mark Carbopol and which consist of polymers of acrylic acid cross-linked with polyallyl sucrose or polyallyl pentaeythritol, for example, Carbopol 934, 940 and 950.

The viscosity of the final composition (Brookfield RVT, Spindle 5, 50 rpm, 25°C, neat) is preferably at least 500 cps, more preferably from 1,000 to 10,000 cps, especially from 2,000 to 6,000 cps.

The cleansing compositions can optionally include a skin moisturizer which is soluble in the cleansing composition matrix. The preferred level of moisturizer is from 0.5% to 20% by weight. In preferred embodiments, the moisturizer is selected from:
1. water-soluble liquid polyols;
2. essential amino acid compounds found naturally occurring in the stratum corneum of the skin; and
3. water-soluble nonpolyol nonocclusives and mixtures thereof.

Some examples of more preferred nonocclusive moisturizers are glycerine, polyethylene glycol, propylene glycol, sorbitol, polyethylene glycol and propylene glycol ethers of methyl glucose (e.g. methyl glucam-20), polyethylene glycol and propylene glycol ethers of lanolin alcohol (e.g. Solulan-75), sodium pyrrolidone carboxylic acid, lactic acid, urea, L-proline, guanidine, pyrrolidone, hydrolyzed protein and other collagen-derived proteins, aloe vera gel and acetamide MEA and mixtures thereof. Of the above, glycerine is highly preferred.

A number of additional optional materials can be added to the cleansing compositions. Such materials include proteins and polypeptides and derivatives thereof; water-soluble or solubilizable preservatives such as DMDM Hydantoin, Germall 115, methyl, ethyl, propyl and butyl esters of hydroxybenzoic acid, EDTA, Euxyl (RTM) K400, Bronopol (2-bromo-2-nitropropane-1,3-diol), sodium benzoate and 2-phenoxyethanol; other moisturizing agents such as hyaluronic acid, chitin, and starch-grafted sodium polyacrylates such as Sanwet (RTM) IM-1000, IM-1500 and IM-2500 available from Celanese Superabsorbent Materials, Portsmith, VA, USA and described in US-A-4,076,663; solvents such as hexylene glycol and propylene glycol; anti-bacterial agents such as Oxeco (phenoxy isopropanol); low temperature phase modifiers such as ammonium ion sources (e.g. NH₄ Cl); viscosity control agents such as magnesium sulfate and other electrolytes; colouring agents; pearlescers and opacifiers such as ethylene glycol distearate, TiO₂ and TiO₂-coated mica; perfumes and perfume solubilizers; and zeolites such as Valfour BV400 and derivatives thereof and Ca²⁺/Mg²⁺ sequestrants such as polycarboxylates, amino polycarboxylates, polyphosphates, polyhosphonates, amino polyphosphonates and gluconates etc. Water is also present at a level preferably of from 45 % to 92% preferably at least 60% by weight of the compositions herein.

The pH of the compositions is preferably from about 4 to about 8.

The invention is illustrated by the following non-limiting examples.

In the examples, all concentrations are on a 100% active basis and the abbreviations have the following designation:

| | |
|---|---|
| Amphoteric | Empigen CDL 60 - an aqueous mixture of 23.5% cocoamphoacetate (in which R₁ is coconut alkyl, R₂ is H, and Z is CO₂Na) and 1.35% cocoamphodiacetate (in which R₁ is coconut alkyl, R₂ is CH₂CO₂Na and Z is CO₂Na). |
| Anionic 1 | Sodium laureth-2 sulfate |
| Anionic 2 | Magnesium sodium laureth 3.6 sulfate |
| Solan | Solan (RTM) E (PEG 55 lanolin) |
| Shea | Hydrosoluble Shea Butter (PEG 75) |
| Crovol | Crovol (RTM) EP 70 (PEG 60 evening primrose triglycerides) |
| GA | Polyhydroxy fatty acid amide of formula VII in which R₈ is C₁₁-C₁₇ alkyl, R₉ is methyl, and Z₂ is CH₂(CHOH)₄CH₂OH |
| DEA | Coconut diethanolamide |
| Betaine | Cocoamidopropyldimethylcarboxymethyl betaine |
| Polymer | Polymer JR-400 - hydroxyethylcellulose reacted with epichlorohydrin and quaternized with trimethylamine, m.wt. 4 x 10⁶ |
| Preservative | DMDM Hydantoin |
| Pearlescer | Ethyleneglycoldistearate/emulsifier mixture |
| Oil | Jojoba Oil |
| Softigen 767 | PEG(6) caprylic/capryl glycerate |
| Mg | Magnesium sulfate heptahydrate |

### Examples I to VII

The following are personal cleansing compositions in the form of shower gel or bath foam products and which are representative of the present invention:

| | I | II | III | IV | V | VI | VII |
|---|---|---|---|---|---|---|---|
| Amphoteric | - | 3.0 | - | 5.0 | - | 5.0 | 5.0 |
| Anionic 1 | 12.0 | 3.0 | 6.0 | 4.0 | 13.0 | 10.0 | 10.0 |
| Anionic 2 | - | 9.0 | 4.0 | 6.0 | - | - | - |
| Solan | 2.0 | 3.0 | 2.0 | 1.0 | - | 2.0 | 3.0 |
| Croval | 2.0 | - | 1.0 | - | - | 3.0 | 1.0 |
| Shea | 2.0 | 2.0 | - | 3.0 | 4.0 | - | - |
| GA | - | - | - | - | 2.0 | - | 2.0 |
| DEA | - | - | - | - | - | - | 1.0 |
| Betaine | 4.0 | 2.0 | 3.0 | - | 3.0 | 2.5 | 2.0 |
| Polymer | - | 0.1 | - | 0.2 | - | - | 0.2 |
| Softigen 767 | - | - | - | - | - | 1.0 | - |
| Oil | - | - | - | - | - | 1.0 | 2.0 |
| Preservative | 0.15 | 0.15 | 0.15 | 0.15 | 0.15 | 0.2 | 0.2 |
| Pearlescer | - | - | - | 1.0 | 1.0 | 2.0 | 1.0 |
| Perfume | 1.5 | 1.5 | 1.5 | 1.5 | 1.5 | 1.5 | 1.5 |
| Glycerine | - | - | - | - | - | - | 3.0 |
| Mg | 1.0 | - | - | - | 2.0 | - | - |
| Sodium Citrate 2.0 | | 3.0 | 3.0 | 2.0 | 3.0 | 2.0 | 4.0 |
| Water | ----------------- to 100 --------------- | | | | | | |

Compositions I to VII are prepared by forming a surfactant phase A at room temperature containing a portion of the water, the anionic, amphoteric and oil-derived nonionic surfactants and the remaining water-soluble, oil-insoluble ingredients. In compositions which comprise water-insoluble ingrediants an oil phase B is formed from these oil-soluble ingredients which is then admixed with A at about 40-70°C and cooled to ambient temperature. Finally the remaining water, preservative and perfume are added.
Finally, the compositions have a viscosity (Brookfield RVT, Spindle 5, 50 rpm, 25°C, neat) in the range from 1,000 to 10,000 cps.

The products provide excellent in-use and efficacy benefits including mildness, skin feel during and after use, skin conditioning, stability, cleansing, lathering and rinsibility.

## Claims

1. A personal cleansing composition for personal cleansing of the skin comprising:
(a) from 10% to 50% by weight of one or more surfactants selected from anionic, zwitterionic and/or amphoteric surfactants and mixtures thereof;
(b) from 0.1 % to 20 % by weight of a soluble or dispersible nonionic surfactant selected from ethoxylated animal and vegetable oils and fats and mixtures thereof;
(c) from 1.5% to 10% by weight of citric acid or water soluble citrate salt or mixture thereof; and,
(d) up to 3% by weight of perfume or cosmetic oil, and
(e) water.

2. A composition according to Claim 1 wherein the composition has a viscosity (Brookfield RVT, Spindle 5, 50 rpm, 25°C, neat) in the range from 1,000 to 10,000 cps.

3. A composition according to Claim 1 or 2 wherein the oil derived nonionic surfactant comprises one or more ethoxylated oil or fat having the formula (V) 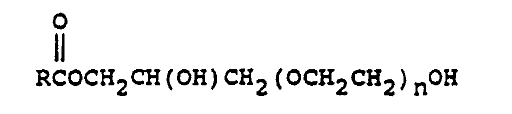 wherein n is from 5 to 200, preferably from 20 to 100, more preferably from 30 to 85, and wherein R comprises an aliphatic radical having an average from 5 to 20 carbon atoms, preferably from 9 to 20 carbon atoms, more preferably from 11 to 18 carbon atoms, most preferably from 12 to 16 carbon atoms.

4. A composition according to Claims 1 to 3 comprising from 2% to 16%, preferably from 3% to 12%, by weight of the oil derived nonionic surfactant.

5. A composition according to Claims 1 to 4 whereia the citric acid or water soluble citrate salt or mixture thereof is at a level from 1.5% to 8%, preferably from 2% to 6%.

6. A composition according to any of Claims 1 to 5 wherein the weight ratio of oil derived nonionic surfactant: citric acid or water soluble salt thereof is in the range from 1:2 to 3:1.

7. A composition according to any of Claims 1 to 6 wherein the oil derived nonionic surfactant and citric acid/acid salt together comprise from 1.5% to 20%, preferably from 2% to 15% most preferably from 2.5% to 10% by weight of the composition.

8. A composition according to Claims 1 to 7 comprising a mixture of anionic with zwitterionic and/or amphoteric surfactants.

9. A composition according to Claims 1 to 8 comprising from 1% to 15%, preferably from 2% to 13% by weight of each of the anionic surfactant and zwitterionic and/or amphoteric surfactant.

10. A composition according to Claims 1 to 9 wherein the anionic surfactant is selected from ethoxylated alkyl sulfates, alkyl glyceryl ether sulfonates, methyl acyl taurates, fatty acyl glycinates, alkyl ethoxy carboxylates, N-acyl glutamates, acyl isethionates, alkyl sulfosuccinates, alpha-sulfonated fatty acids, their salts and/or their esters, alkyl phosphate esters, ethoxylated alkyl phosphate esters, acyl sarcosinates and fatty acid/protein condensates, and mixtures thereof.

11. A composition according to any of Claims 1 to 10 wherein the anionic surfactant comprises an ethoxylated C ₈-C₂₂ alkyl sulfate.

12. A composition according to any of Claims 1 to 11 wherein the amphoteric surfactant is selected from:
(a) imidazolinium derivatives of formula (I) 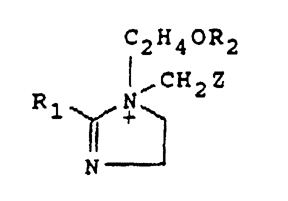 wherein R₁ is C₇-C₂₂ alkyl or alkenyl, R₂ is hydrogen or CH₂Z, each Z is independently CO₂M or CH₂CO₂M, and M is H, alkali metal, alkaline earth metal, ammonium or alkanolammonium; and/or ammonium derivatives of formula (II) 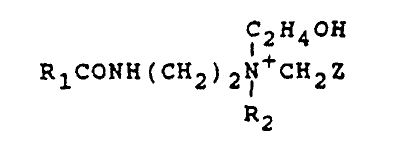 wherein R₁, R₂ and Z are as defined above:
(b) aminoalkanoates of formula (III)
R₁NH(CH₂)ₙCO₂M
and iminodialkanoates of formula (IV)
R₁N[(CH₂)ₘCO₂M]₂
wherein n and m are numbers from 1 to 4, and R₁ and M are independently selected from the groups specified in (a) above; and
(c) mixtures thereof.

13. A composition according to Claim 12 wherein the amphoteric is selected from the imidazolinium derivatives of formula I and/or ammonium derivatives of formula II.

14. A composition according to any of Claims 1 to 13 wherein the weight ratio of anionic surfactant:zwitterionic and/or amphoteric surfactant is in the range from 1:2 to 6:1.

15. A composition according to any of Claims 1 to 14 wherein the anionic surfactant, zwitterionic and/or amphoteric surfactant together comprise from 8% to 35%, preferably from 10% to 30% by weight of the composition.

16. A composition according to any of Claims 1 to 15 which additionally comprises from 0.1 % to 20% by weight of an auxilliary nonionic surfactant selected from C12-C14 fatty acid mono-and diethanolamides and polyhydroxy fatty acid amide surfactants.

17. A composition according to any of Claims 1 to 16 which is in the form of an optically clear solution or microemulsion.

18. A composition according to any of Claims 1 to 17 wherein the weight ratio of soluble nonionic surfactant: cosmetic oil (if present) is at least 1:2.

19. A composition according to any of Claims 1 to 18 additionally comprising from 0.01% to 5%, preferably from 0.04% to 2% and more preferably from 0.05% to 1% of a cationic or nonionic polymeric skin or hair conditioning agent, selected from cationic and nonionic polysaccharides; cationic and nonionic homopolymers and copolymers derived from acrylic and/or methacrylic acid, cationic and nonionic cellulose resins; cationic copolymers of dimethyldiallylammonium chloride and acrylic acid; cationic homopolymers of dimethyldiallylammonium chloride; cationic polyalkylene and ethoxypolyalkylene imines; quaternized silicones, and mixtures thereof.

20. A composition according to any of Claims 1 to 19 additionally comprising moisturiser selected from glycerin, polyethylene glycol, propylene glycol, sorbitol, polyethylene glycol and polypropylene glycol ethers of methyl glucose, sodium pyrrolidone carboxylic acid, lactic acid, L-proline and mixtures thereof.

## Patentansprüche

1. Körperreinigungszusammensetzung zur Körperreinigung der Haut, umfassend:
(a) 1 Gew.-% bis 50 Gew.-% eines oder mehrerer Tenside, ausgewählt aus anionischen, zwitterionischen und/oder amphoteren Tensiden sowie Mischungen davon:
(b) 0.1 Gew.-% bis 20 Gew.-% eines löslichen oder dispergierbaren nichtionischen Tensids, ausgewählt aus ethoxylierten tierischen und pflanzlichen Ölen und Fetten sowie Mischungen davon;
(c) 1.5 Gew.-% bis 10 Gew.-% Citronensäure oder wasserlöslichem Citratsalz oder eine Mischung davon: und
(d) bis zu 3 Gew.-% Duftstoff oder kosmetisches Öl, und
(e) Wasser.

2. Zusammensetzung nach Anspruch 1, worin die Zusammensetzung eine Viskosität (Brookfield RVT. Spindel 5. 50 U/min. 25°C, unverdünnt) in dem Bereich von 1.000 bis 10.000 cps aufweist.

3. Zusammensetzung nach Anspruch 1 oder 2, worin das ölabgeleitete nichtionische Tensid ein oder mehrere ethoxylierte Öle oder Fette mit der Formel (V) umfasst 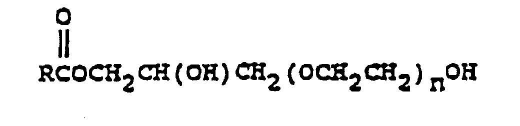 worin n von 5 bis 200, bevorzugt von 20 bis 100. ganz besonders bevorzugt von 30 bis 85, reicht und worin R einen aliphatischen Rest mit im Mittel 5 bis 20 Kohlenstoffatomen, bevorzugt 9 bis 20 Kohlenstoffatomen, besonders bevorzugt 11 bis 18 Kohlenstoffatomen, am meisten bevorzugt 12 bis 16 Kohlenstoffatomen. umfasst.

4. Zusammensetzung nach den Ansprüchen 1-3, umfassend 2 Gew.-% bis 16 Gew.-%, bevorzugt 3 Gew.-% bis 12 Gew.-%, ölabgeleitetes nichtionisches Tensid.

5. Zusammensetzung nach den Ansprüchen 1-4, worin die Citronensäure oder das wasserlösliche Citratsalz oder die Mischung davon in einem Anteil von 1.5% bis 8%, bevorzugt von 2% bis 6%, vorliegt.

6. Zusammensetzung nach mindestens einem der Ansprüche 1-5, worin das Gewichtsverhältnis des ölabgeleiteten nichtionischen Tensids: Citronensäure oder wasseriöslichem Salz davon in dem Bereich von 1:2 bis 3:1 liegt.

7. Zusammensetzung nach mindestens einem der Ansprüche 1-6, worin das ölabgeleitete nichtionische Tensid und Citronensäure/Säuresalz zusammen 1.5 Gew.-% bis 20 Gew.-%, bevorzugt 2 Gew.-% bis 15 Gew.-%, ganz besonders bevorzugt 2,5 Gew.-% bis 10 Gew.-%, der Zusammensetzung umfassen.

8. Zusammensetzung nach den Ansprüchen 1-7, umfassend eine Mischung von anionischen mit zwitterionischen und/oder amphoteren Tensiden.

9. Zusammensetzung nach den Ansprüchen 1-8, umfassend 1 Gew.-% bis 15 Gew.-%. bevorzugt 2 Gew.-% bis 13 Gew.-%, eines jeden des anionischen Tensids und zwitterionischen und/oder amphoteren Tensids:

10. Zusammensetzung nach den Ansprüchen 1-9. worin das anionische Tensid ausgewählt ist aus ethoxylierten Alkylsulfaten, Alkylglycerylethersulfonaten, Methylacyltaurate Fettacylglycinaten. Alkylethoxycarboxylaten, N-Acylglutamaten. Acylisethionaten, Alkylsulfosuccinaten, alpha-sulfonierten Fettsäuren, deren Salzen und/oder deren Estern. Alkylphosphatestern, ethoxylierten Alkylphosphatestern, Acylsarcosinaten und Fettsäure/Proteinkondensaten, sowie Mischungen davon.

11. Zusammensetzung nach mindestens einem der Ansprüche 1-10, worin das anionische Tensid ein ethoxyliertes C₈-C₂₂-Alkylsulfat umfasst.

12. Zusammensetzung nach mindestens einem der Ansprüche 1-11. worin das amphotere Tensid ausgewählt ist aus:
(a) Imidazolinium-Derivaten der Formel (I) 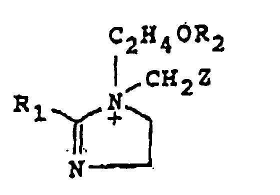 worin R₁ C₇-C₂₂-Alkyl oder Alkenyl ist. R₂ Wasserstoff oder CH₂Z ist, Z jeweils unabhängig voneinander CO₂M oder CH₂CO₂M ist, und M H ist. ein Alkalimetall, ein Erdalkalimetall. Ammonium oder Alkanolammonium;
und/oder Ammoniumderivaten der Formel (II) 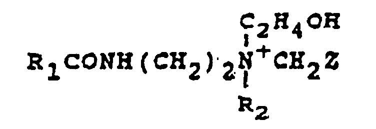 wort R₁, R₂ und Z wie oben definiert sind:
(b) Amlnoalkanoaten der Formel (III)
R₁NH(CH₂)ₙCO₂M
und Iminodialkanoaten der Formel (IV)
R₁N[(CH₂)ₘCO₂M]₂
worin n und m Zahlen von 1 bis 4 sind und R₁ und M unabhängig voneinander ausgewählt sind aus den Gruppen, wie sie in (a) oben spezifiziert sind: und
(c) Mischungen davon.

13. Zusammensetzung nach Anspruch 12, worin das amphotere Tensid ausgewählt ist aus Imidazoliniumderivaten der Formel (I) und/oder Ammoniumderivaten der Formel (II).

14. Zusammensetzung nach mindestens einem der Ansprüche 1-13, worin das Gewichtsverhältnis von anionischem Tensid: zwitterionischem und/oder amphoterem Tensid in dem Bereich von 1:2 bis 6:1 liegt.

15. Zusammensetzung nach mindestens einem der Ansprüche 1-14, worin das anionische Tensid, zwitterionische und/oder amphotere Tensid zusammen 8 Gew.-% bis 35 Gew.-%. bevorzugt 10 Gew.-% bis 30 Gew.-%, der Zusammensetzung umfassen.

16. Zusammensetzung nach mindestens einem der Ansprüche 1-15, welche zusätzlich 0.1 Gew.-% bis 20 Gew.-% eines hilfsweisen nichtionischen Tensids, ausgewählt aus C₁₂-C₁₄-Fettsäuremono- und -diethanolamiden und Polyhydroxyfettsäureamid-Tensiden, umfasst.

17. Zusammensetzung nach mindestens einem der Ansprüche 1-16, welche In der Form einer optisch klaren Lösung oder Mikroemulsion vorliegt.

18. Zusammensetzung nach mindestens einem der Ansprüche 1-17, worin das Gewichtsverhältnis von lüslichem nichtionischen Tensid: kosmetischem Öl, wenn anwesend, mindestens 1:2 beträgt.

19. Zusammensetzung nach mindestens einem der Ansprüche 1-18, zusätzlich umfassend 0.01% bis 5%, bevorzugt 0.04% bis 2% und ganz besonders bevorzugt 0.05% bis 1%, eines kationischen oder nichtionischen polymeren Haut- oder Haarkonditionierungsmittels, ausgewählt aus kationischen und nichtionischen Polysacchariden: kationischen und nichtionischen Homopolymeren und Copolymeren. abgeleitet von Acryl- und/oder Methacrylsäure, kationischen und nichtionischen Celluloseharzen; kationischen Copolymeren von Dimethyldiallylammoniumchlorid und Acrylsäure: kationischen Homopolymeren von Dtmethyldiallylammoniumchlorid; kationischen Polyalkylen- und Ethoxypolyalkyleniminen: quaternisierten Silikonen, sowie Mischungen davon.

20. Zusammensetzung nach mindestens einem der Ansprüche 1-19, zusätzlich umfassend Feuchthaltemittel, ausgewählt aus Glycerin, Polyethylenglykol, Propylenglykol, Sorbit, Polyethylenglykol- und Polypropylenglykolether von Methylglucose, Natriumpyrrolidoncarbonsäure, Milchsäure, L-Prolin und Mischungen davon.

## Revendications

1. Composition d'hygiène corporelle pour la toilette de la peau comprenant :
(a) 10% à 50% en poids d'un ou plusieurs tensioactifs choisis parmi les tensioactifs anioniques, zwittérioniques et/ou amphotères et leurs mélanges ;
(b) 0,1% à 20% en poids d'un tensioactif non ionique soluble ou dispersible choisi parmi des huiles et des graisses animales et végétales éthoxylées et leurs mélanges ;
(c) 1,5% à 10% en poids d'acide citrique ou d'un sel de citrate hydrosoluble ou leur mélange ; et
(d) jusqu'à 3% en poids de parfum ou d'huile cosmétique, et
(e) de l'eau.

2. Composition selon la composition selon la revendication 1, dans laquelle la composition a une viscosité (Brookfield RVT, aiguille 5, 50 tr/min, 25°C, pur) dans la plage de 1000 à 10 000 cP.

3. Composition selon la revendication 1 ou 2, dans laquelle le tensioactif non ionique dérivé d'huile comprend une ou plusieurs huiles ou graisses éthoxylées avant la formule (V) 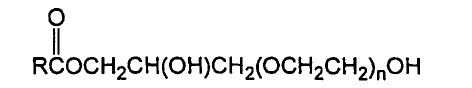 dans laquelle n vaut 5 à 200, de préférence 20 à 100, mieux encore 30 à 85, et dans laquelle R comprend un radical aliphatique ayant une moyenne de 5 à 20 atomes de carbone, de préférence 9 à 20 atomes de carbone, mieux encore 11 à 18 atomes de carbone, bien mieux encore 12 à 16 atomes de carbone.

4. Composition selon la revendication 1 à 3 comprenant 2% à 16%, de préférence 3% à 12%, en poids du tensioactif non ionique dérivé de l'huile.

5. Composition selon les revendications 1 à 4, dans laquelle l'acide citrique ou un sel citrate hydrosoluble ou leur mélange est en une quantité de 1,5% à 8%, de préférence 2% à 6%.

6. Composition selon l'une quelconque des revendications 1 à 5, dans laquelle le rapport en poids tensioactif non ionique dérivé d'huile : acide citrique ou son sel hydrosoluble est dans la plage de 1 : 2 à 3 : 1.

7. Composition selon l'une quelconque des revendications 1 à 6, dans laquelle le tensioactif non ionique dérivé d'huile et l'acide citrique/sel d'acide citrique constituent ensemble 1,5% à 20%, de préférence 2% à 15%, mieux encore 2,5% à 10% en poids de la composition.

8. Composition selon les revendications 1 à 7, comprenant un mélange de tensioactifs anioniques avec des tensioactifs zwittérioniques et/ou amphotères.

9. Composition selon les revendications 1 à 8 comprenant 1% à 15%, de préférence 2% à 13% en poids de chacun du tensioactif anionique et des tensioactifs zwittérioniques et/ou amphotères.

10. Composition selon les revendications 1 à 9, dans laquelle le tensioactif anionique est choisi parmi les alkylsulfates éthoxylés, les alkylglycéryléthersulfonates, les méthylacyltaurates, des acylglycinates gras, les alkylcarboxylates éthoxylés, les N-acylglutamates, les acyliséthionates, les alkylsulfosuccinates, les acides gras alpha-sulfonés, leurs sels et/ou leurs esters, les esters d'alkylphosphates, les esters d'alkylphosphates éthoxylés, les acylsarcosinates et les condensats acide gras/protéine, et leurs mélanges.

11. Composition selon l'une quelconque des revendications 1 à 10, dans laquelle le tensioactif anionique comprend un alkylsulfate en C₈-C₂₂ éthoxylé.

12. Composition selon l'une quelconque des revendications 1 à 11, dans laquelle le tensioactif amphotère est choisi parmi :
(a) des dérivés d'imidazolinium de formule (I) 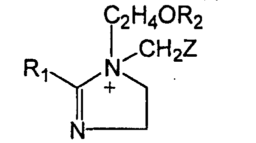 dans laquelle R₁ est un groupe alkyle ou alcényle en C₇-C₂₂, R₂ est l'hydrog ou CH₂Z, chaque Z est indépendamment CO₂M ou CH₂CO₂M, et M est H, un métal alcalin, un métal alcalino-terreux, un ammonium ou un alcanolammonium ; et/ou des dérivés de formule (II) 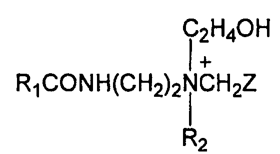 dans laquelle R₁, R₂ et Z sont tels que définis ci-dessus ;
(b) des aminoalcanoates de formule (III)
R₁NH(CH₂)ₙCO₂M
et des iminodialcanoates de formule (IV)
R₁N[(CH₂)ₘCO₂M]₂
dans laquelle n et m sont des nombres de 1 à 4, et R₁ et M sont choisis indépendamment parmi les groupes spécifiés dans (a) ci-dessus ; et
(c) leurs mélanges.

13. Composition selon la revendication 12, dans laquelle l'amphotère est choisi parmi les dérivés d'imidazolinium de formule I et/ou les dérivés d'ammonium de formule II.

14. Composition selon l'une quelconque des revendications 1 à 13, dans laquelle le rapport en poids tensioactif anionique : tensioactif zwittérionique et/ou amphotère est dans la plage de 1 : 2 à 6 : 1.

15. Composition selon l'une quelconque des revendications 1 à 14, dans laquelle le tensioactif anionique, le tensioactif zwittérionique et/ou amphotère constituent ensemble 8% à 35%, de préférence 10% à 30% en poids de la composition.

16. Composition selon l'une quelconque des revendications 1 à 15, qui comprend en outre 0,1% à 20% en poids d'un tensioactif non ionique auxiliaire choisi parmi les tensioactifs mono- et diéthanolamides d'acides gras en C₁₂-C₁₄ et amides d'acide gras polyhydroxylés.

17. Composition selon l'une quelconque des revendications 1 à 16, qui est sous forme d'une solution ou d'une microémulsion transparente optiquement.

18. Composition selon l'une quelconque des revendications 1 à 17, dans laquelle le rapport en poids tensioactif non ionique soluble : huile cosmétique (si elle est présente) est d'au moins environ 1 : 2.

19. Composition selon l'une quelconque des revendications 1 à 18, comprenant en outre 0,01% à 5%, de préférence 0,04% à 2%, et mieux encore 0,05% à 1% d'un agent de conditionnement de la peau ou des cheveux cationique ou non ionique, choisi parmi les polysaccharides cationiques et non ioniques; les homopolymères et copolymères cationiques et non ioniques dérivés d'acide acrylique et/ou méthacrylique, les résines de cellulose cationiques et non ioniques; les copolymères cationiques de chlorure de diméthyldiallylammonium et d'acide acrylique ; les homopolymères cationiques de chlorure de diméthyldiallylammonium ; les polyalkylèneimines et les polyalkylèneimines éthoxylées cationiques ; les silicones quaternisées, et leurs mélanges.

20. Composition selon l'une quelconque des revendications 1 à 19, comprenant en outre des hydratants choisis parmi la glycérine, le polyéthylèneglycol, le propylèneglycol, le sorbitol, le polyéthylèneglycol et des éthers de polypropylèneglycol et de méthylglucose, le sel de sodium d'acide pyrrolidone-carboxylique, l'acide lactique, la L-proline et leurs mélanges.
